# EUROPEAN PATENT APPLICATION

(11) **EP 4 656 175 A1**
(43) Date of publication of application: **03.12.2025**
(21) Application number: 23918646.3
(22) Date of filing: 21.12.2023
(51) Int. Cl.: A61K 6/887, A61K 6/818, A61K 6/831

(54) **DENTAL CEMENT AND DENTAL CEMENT PREPARATION KIT**

(30) Priority: 23.01.2023 JP 2023008042
(71) Applicant: Tokuyama Dental Corporation, Tokyo 110-0016 (JP)
(72) Inventor: HONDA, Akane, Tokyo 110-0016 (JP); SUZUKI, Hiroki, Tokyo 110-0016 (JP); KISHI, Hiroto, Tokyo 110-0016 (JP)
(74) Representative: Lorenz & Kollegen
(86) International application number: PCT/JP2023/045965
(87) International publication number: WO 2024/157692

(57) **Abstract**

[Object] To provide dual cure cement with a wide range of a light irradiation amount that allows a favorable semi-cured state in which the excess cement can be easily removed to be maintained for several minutes, the cured body of the dual cure cement having a significantly higher strength than that of the existing dual cure cement.

[Solving Means] Dental cement includes: a composition that includes a polymerizable monomer, a filler, a chemical polymerization initiator, and a photopolymerization initiator, the dental cement being characterized in that a specific tertiary aromatic amine that inhibits photopolymerization, such as N,N-di(1-hydroxyethyl)-p-toluidine, is not substantially included, the chemical polymerization initiator includes a thiourea compound having a specific structure, such as N-benzoylthiourea: 0.4 to 1.5 parts by mass, the photopolymerization initiator includes an α-diketone (d1): 0.3 to 0.8 parts by mass and a photopolymerization accelerator: 0.3 to 0.8 parts by mass, and a ratio of a content: x (parts by mass) of the (d1) to a content: y (parts by mass) of the (c2) is 0.3 to 1.9.

## Description

### Technical Field

The present invention relates to dental cement and a dental cement preparation kit.

### Background Art

As cementing materials (dental cement) of a restoration such as an inlay, an onlay, a laminate veneer, and a jacket crown in dental treatment, not only a material that causes light to be transmitted therethrough, such as ceramics and composite resins, but also dual cure cement that is formed of a material that does not allow light to be transmitted therethrough and can also be applied to the above restoration is used.

The above dual cure cement is formed of a paste-like curable composition having high fluidity, which includes a polymerizable monomer, a filler, a chemical polymerization initiator, and a photopolymerization initiator. In the case where this dual cure cement is used to adhere a tooth substance and a tooth crown restoration material to each other, an excess amount of dental cement is applied to the restoration material and the tooth crown restoration material is pressed against the tooth substance. At this time, it is necessary to remove the excess dental cement (referred to also as "excess cement") that has protruded from a bonding portion between the tooth substance and the tooth crown restoration material, which is called a margin portion, during the above pressing. This removal of the excess cement is typically performed by making the excess cement be in a semi-cured state (state in which curing has progressed to the point where fluidity has largely ceased) and scraping it off using a dental short needle or the like from the viewpoint of the operability. Note that removing excess cement from the oral cavity is a delicate work that requires precision and takes several minutes in some cases, and it is desirable to maintain the semi-cured state during the work.

In the case of using the dual cure cement, the removal may be started after the excess cement reaches the above semi-cured state through chemical polymerization without light irradiation, but it is common to irradiate the excess cement with light in an appropriate amount after the above pressing to make the excess cement be in the semi-cured state from the viewpoint of time servings. As dual cure cement that can be easily removed when the excess cement is made in the semi-cured state by such a method, the following is known.

That is, Patent Literature 1 discloses the dual cure cement that includes (A) a polymerizable monomer, (B) an α-diketone compound, (C) a composition obtained by combining three types of tertiary aromatic amines i) to iii) each having a specific structure, (D) a filler, and (E) a peroxide. Here, Patent Literature 1 describes that each of the three types of tertiary aromatic amines constituting the above (C) functions as a co-catalyst for α-diketone, the type i) of these has a function of promoting photopolymerization, and the other two types ii) and iii) have a function of preventing the time during which the semi-cured state can be maintained from becoming shorter due to the user of the type i). Then, according to Patent Literature 1, when the excess cement generated by pressing the above dual cure cement is irradiated with light with a light intensity: 380 to 420 mW/cm² and an irradiation distance: approximately 1 cm for 2 to 4 seconds, it reaches a semi-cured state (suitable for removal work), and this state can be maintained for 2 minutes or more (corresponding to the time until the effect of cuing due to chemical polymerization begins to appear).

Further, Patent Literature 2 discloses, as a dental adhesive material kit having excellent removability when removing excess cement in a semi-cured state temporarily irradiated with light by a light irradiator, and appropriate strength, a dental adhesive material kit that includes a dental waterbased adhesive (A) and a dental curable composition (B), in which a difference: (t2-t1) between the polymerization start time of (B): t2 (min) and the polymerization start time of (A) and (B) when being in contact with each other: t1 (min) is set to 3 (min) or less. Then, according to Patent Literature 2, the above (A) means a surface treatment agent (primer) for tooth surfaces and the (B) means resin cement that adheres a tooth substance and a prosthesis when it is used in a dental cement kit. Further, pressing is performed using one including a radically polymerizable monomer (a) having an acidic group, a radically polymerizable monomer (b-1) having no amino group and no acidic group, a polymerization accelerator (c), and water (d) as the above (A) and one including a radically polymerizable monomer (b) having no acidic group, a polymerization accelerator (c), a chemical polymerization initiator (f), a photopolymerization initiator (g), and a filler (h) as the above (B), and when the excess cement after one minute was irradiated with light using a dental LED light irradiator in a standard mode at a rate of 10 seconds per rotation, the excess cement could be easily removed in one piece.

### Citation List

### Patent Literature

Patent Literature 1: Japanese Patent No. 5773676
Patent Literature 2: Specification of WO 2020/111142

### Disclosure of Invention

### Technical Problem

In the case where the excess cement is irradiated with light to be in the semi-cured state, the state of the excess cement after the light irradiation, such as fluidity, depends on the light irradiation condition. In order to achieve the state suitable for removal work, it is necessary to control the light irradiation state. The conditions adopted in Patent Literature 1 were evaluated in simulated tests using cow teeth and serve as a general guidance. However, in actual treatment, it is difficult to bring the irradiation window of the light irradiator close to the excess cement in some cases, resulting in light irradiation with a weaker intensity. In addition, due to blurring and the like, the irradiation time (the time during which the irradiation target part is actually irradiated with light) is reduced in some cases. Then, in such a case, a desired semi-cured state cannot be achieved. For example, the present inventors evaluated the removability of excess cement by irradiating the cement in Patent Literature 1 with light using an LED light irradiator at 300 mW/cm², and found that the cement did not reach a semi-cured state under the condition of light irradiation for one second and it was difficult to remove the excess cement in one piece (see Comparative Example 9 described below).

Further, in dental cement, it is desirable that the dental cement present in the bonding portion is completely cured to form a cured body with a high strength from the viewpoint of adhesive durability. That is, when the strength of the cement cured body is low, the restoration is more likely to become detached. The cured bodies of the existing dual cure cement disclosed in Patent Literatures 1 and 2 has a bending strength of approximately 100 to 130 (MPa), leaving room for improvement.

In this regard, the present invention aims to provide dual cure cement with a wide range of a light irradiation amount (product of irradiation time and light intensity) that allows, when irradiating the excess cement generated by pressing with light to make it be in the semi-cured state, a favorable semi-cured state in which the excess cement can be easily removed to be maintained for several minutes, the excess cement being removable with a favorable operability even with a small amount of irradiation, the cured body of the dual cure cement having a significantly higher strength than that of the existing dual cure cement.

### Means for solving the Problem

The present invention solves the above object, and dental cement according to a first embodiment of the present invention (hereinafter, referred to also as "dental cement according to the present invention") includes: a composition that includes a polymerizable monomer (A), a filler (B), a chemical polymerization initiator (C), and a photopolymerization initiator (D). A blending amount of the filler (B) is 50 to 500 parts by mass with respect to 100 parts by weight of the polymerizable monomer (A). The dental cement according to the present invention does not substantially include a tertiary aromatic amine represented by the following general formula (1). The chemical polymerization initiator (C) includes a hydroperoxide (c1): 0.2 to 3.5 parts by mass and a thiourea compound (c2) represented by the following general formula (2): 0.4 to 1.5 parts by mass, and the photopolymerization initiator (D) includes an α-diketone (d1): 0.3 to 0.8 parts by mass and a photopolymerization accelerator (d2): 0.3 to 0.8 parts by mass. A ratio of a content (parts by mass) of the α-diketone (d1) to a content (parts by mass) of the thiourea compound (c2) is 0.3 to 1.9. 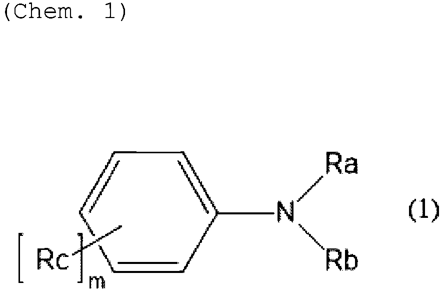 (wherein, Rₐ and R_{b} each represent an alkyl group having 1 to 6 carbon atoms or a substituted alkyl group having 1 to 6 carbon atoms having a hydroxyl group, a nitro group, a sulfonic group, or a halogen atom as a substituent group, which may be different from each other, at least one of Rₐ or R_{b} represents the substituted alkyl group, R_{c} represents an alkyl group having 1 to 4 carbon atoms, and m represents an integer of 0 to 3.) 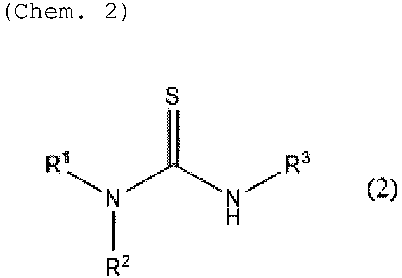 (wherein, R¹, R², and R³ each represent a hydrogen atom, an alkyl group, a cycloalkyl group, an aryl group, a heterocyclic group, an acyl group, an aralkyl group, or an alkenyl group, and R¹ and R² may be bonded to each other to form a ring.)

A second embodiment of the present invention is a dental cement preparation kit (hereinafter, referred to also as a "kit according to the present invention", including: a first agent and a second agent packaged separately, the dental cement according to the above embodiment being prepared by mixing the two agents. The first agent includes the thiourea compound (c2) and the α-diketone (d1). The second agent includes the hydroperoxide (c1).

### Advantageous Effects of Invention

The dental cement according to the present invention is dual cure cement having excellent features that it has a wide range of a light irradiation amount that allows a favorable semi-cured state in which the excess cement can be easily removed to be maintained for several minutes, the excess cement can be easily removed even if the irradiation time is reduced or the light intensity is weakened, and the cured body of the dual cure cement has a high strength.

### Mode(s) for Carrying Out the Invention

### 1. Overview of present invention

As described above, in the dual cure cement disclosed in Patent Literature 1, the removability of excess cement is reduced when the light irradiation amount is small. The present inventors hypothesized that the cause of this might lie in that the "tertiary aromatic amine ii)" that is one of the three types of tertiary aromatic amines forming the tertiary aromatic amine composition (C), i.e., a "tertiary aromatic amine" in which one "phenyl group in which one to three hydrogen atoms may be substituted with an alkyl group having 1 to 4 carbon atoms" and two "substituted or unsubstituted alkyl groups having 1 to 6 carbon atoms" are bonded to a nitrogen atom and at least one of the two "substituted or unsubstituted alkyl groups having 1 to 6 carbon atoms" has a hydroxyl group, a nitro group, a sulfonic group, or a halogen atom as a substituent group, reduced the activity of the photopolymerization initiator, and investigated a system that does not include the above "tertiary aromatic amine ii)". Specifically, Patent Literature 1 describes that in the case where the above "tertiary aromatic amine ii)" is not included (Comparative Example 2), the light curing speed is fast and complete curing is achieved in three seconds of light irradiation. In this regard, the present inventors investigated a system in which the blending amount of the α-diketone (d1) is reduced (in the above Comparative Example, 2 parts by mass of the α-diketone is used with respect to 100 parts by mass of the polymerizable monomer).

As a result, in this system, although the desired semi-cured state could be achieved in some cases even with a small light irradiation amount, the strength of the cured body obtained by photopolymerization alone was reduced, and even with the compensating effect of chemical polymerization, the final strength of the cured body was at the same level as that of the existing one.

In this regard, the present inventors conducted further investigations with the aim of enhancing the strength of the cured body without reducing the activity of the photopolymerization initiator (D), and found that when the specific thiourea compound (c2) is mixed as a so-called polymerization accelerator that functions as a reducing agent of the hydroperoxide (c1), a favorable semi-cured state is achieved in a relatively wide range of the light irradiation amount including a small light irradiation amount even if the blending amount of the α-diketone (d1) is increased (finally to an amount where sufficient light curing occurs), and when the ratio of the blending amount of the specific thiourea compound (c2) and the blending amount of the α-diketone (d1) is within a specific range, the final strength of the cured body is improved, thereby completing the present invention.

The present invention is not limited to any particular theory, but the reason why the above effects can be achieved in the dental cement according to the present invention is presumed to be as follows. That is, it is conceivable that when the specific thiourea compound (c2) and the α-diketone (d1) are mixed at a specific ratio, the above thiourea compound (c2) acts also as a chain transfer agent, and thus, the range of a light irradiation amount in which a semi-cured state is achieved is not narrowed even if the amount of the photopolymerization initiator (D) is increased. Further, it is conceivable that the improvement in the strength of the cement cured body is due to the usability of the photopolymerization initiator (D) in the amount where complete light curing occurs because it is known that when complete curing occurs, the cured body by photopolymerization has a higher strength than the cured body by chemical polymerization (at room temperature).

The dental cement according to the present invention and the dental cement preparation kit according to the present invention will be described below in detail. Note that in this specification, unless otherwise specified, the notation "x to y" using numerical values x and y means "x or more and y or less". In such a notation, in the case where a unit is attached to only the numerical value y, the unit applies also to the numerical value x. Further, in this specification, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

### 2. Details of dental cement

### (1) Overall configuration

The dental cement according to the present invention is dual cure cement, including: a composition that includes a polymerizable monomer (A), a filler (B), a chemical polymerization initiator (C), and a photopolymerization initiator (D), in which the blending amount of the filler(B) is 50 to 500 parts by mass with respect to 100 parts by weight of the polymerizable monomer (A). Then, the dental cement according to the present invention has a great feature that specific ones are used in specific amounts as the chemical polymerization initiator (C) and the photopolymerization initiator (D) and a specific tertiary aromatic amine that reduces the activity of the photopolymerization initiator (D) used is not used.

That is, the composition constituting the dental cement according to the present invention needs to satisfy the following conditions [1] to [4].
[1] A tertiary aromatic amine represented by the following general formula (1) (hereinafter, referred to also as "non-contained aromatic amine") is not substantially included.

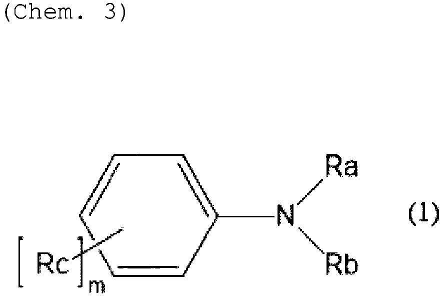

Note that in the above formula (1), Rₐ and R_{b} each independently represent an alkyl group having 1 to 6 carbon atoms or a substituted alkyl group having 1 to 6 carbon atoms having a hydroxyl group, a nitro group, a sulfonic group, or a halogen atom as a substituent group, at least one of Rₐ or R_{b} represents the substituted alkyl group, R_{c} represents an alkyl group having 1 to 4 carbon atoms, and m represents an integer of 0 to 3.

Here, the phrase "not substantially included" means that it is not completely included or it is included in an amount that does not affect the polymerization activity of the photopolymerization initiator (D), and means that the molar ratio N_{AM}/N_{d1} of the content: N_{AM} (mol) of the non-contained aromatic amine to the content: N_{d1} (mol) of the α-diketone (d1) is 0 to 1/500, favorably 0 to 1/1000.

[2] As the chemical polymerization initiator (C), one including the hydroperoxide (c1): 0.2 to 3.5 parts by mass and the thiourea compound (c2) represented by the following general formula (2): 0.4 to 1.5 parts by mass is used.

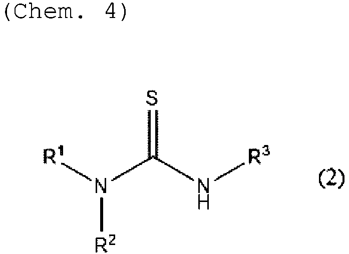

Note that in the above formula (2), R¹, R², and R³ each independently represent a hydrogen atom, an alkyl group, a cycloalkyl group, an aryl group, a heterocyclic group, an acyl group, an aralkyl group, or an alkenyl group, and R¹ and R² may be bonded to each other to form a ring.

[3] As the photopolymerization initiator (D), one including the α-diketone (d1): 0.3 to 0.8 parts by mass and the photopolymerization accelerator (d2): 0.3 to 0.8 parts by mass is used.

[4] The ratio x/y of the content x (parts by mass) of the α-diketone (d1) to the content y (parts by mass) of the thiourea compound (c2) is 0.3 to 1.9.

In the case where these conditions are not satisfied, the effects of the present invention cannot be achieved. For example, in the case where the condition [1] is not satisfied, the removability of the excess cement is reduced when the light irradiation amount is small. Further, in the condition [2], particularly, when the blending amount of the thiourea compound (c2) falls below the lower limit value of the above range, light curing progresses with light irradiation in an extremely short time and the range of a light irradiation amount in which a favorable semi-cured state is achieved becomes extremely narrow. Meanwhile, when the blending amount of the thiourea compound (c2) exceeds the upper limit value, curing becomes insufficient when the light irradiation intensity is weak and the irradiation time is short, and the removability of excess cement is reduced. Further, in the condition [3], a high strength of the cured body cannot be achieved in the case where the blending amount of the α-diketone (d1) falls below the lower limit value of the above range, and the range of a light irradiation amount in which a favorable semi-cured state is achieved becomes narrow similarly to the above in the case where the blending amount of the α-diketone (d1) exceeds the upper limit value. Further, light curing progresses with light irradiation in an extremely short time and the range of a light irradiation amount in which a favorable semi-cured state is achieved becomes extremely narrow in the case where the condition [4] is not satisfied and the ratio x/y is less than the lower limit value, and curing becomes insufficient when the light irradiation amount is small and the removability of excess cement is reduced in the case where the ratio x/y exceeds the upper limit value.

The dental cement according to the present invention is basically the same as the existing dual cure cement except for the above features, those that can be used in the existing dual cure cement can be used as the polymerizable monomer (A) and the filler (B) without any restrictions, and the blending amount of the filler (B) is also within the range of a general blending amount. Further, those that can be used in the existing dual cure cement can be used as the hydroperoxide (c1), the α-diketone (d1), and a photopolymerization accelerator without any particular restrictions. In this regard, the non-contained aromatic amine, which is not substantially included in the dental cement according to the present invention in the condition [1], will be described first, and then, details of each component used in the dental cement according to the present invention will be described below.

### (2) Details of each component

### (a) Non-contained aromatic amine

The non-contained aromatic amine is the compound represented by the general formula (1) and corresponds to the tertiary aromatic amine ii) used in the dual cure dental cement disclosed in Patent Literature 1. It is conceivable that the non-contained aromatic amine has a function of reducing the activity of the photopolymerization initiator, and including this component makes it difficult to achieve the effects of the present invention. For this reason, the dental cement according to the present invention does not substantially include the non-contained aromatic amine. That is, regarding the content: N_{AM} (mol) of the non-contained aromatic amine included in the dental cement according to the present invention, the molar ratio N_{AM}/N_{d1} of the content: N_{AM} (mol) to the content: Nd1 (mol) of the component (d1) is 1/500 or less, favorably 1/1000 or less, and most favorably 0 (the non-contained aromatic amine is not completely included).

Examples of the non-contained aromatic amine include 2,2-[3-(methylphenyl)imino]bisethanol acetate, 1,1-[(4-methylphenyl)imino]bis(2-propanol), N,N-di(1-hydroxyethyl)-p-toluidine, N,N-bis(2,2,2-trifluoroethyl)-p-toluidine, N,N-di(1-hydroxyethyl)-p-toluidine, N,N-di(2-hydroxypropyl)-p-toluidine, N-(1-cyanoethyl)-N-(1-acetoxyethyl)-m-toluidine, and N,N-di(1-chloroethyl)-p-toluidine.

Note that the non-contained aromatic amine is used as a reducing agent in a redox chemical polymerization initiator in some cases. However, in order to satisfy the condition [1], the chemical polymerization initiator (C) used in the dental cement according to the present invention, a chemical polymerization initiator that substantially includes the non-contained aromatic amine as a reducing agent is not used.

### (b) Polymerizable monomer (A)

As the polymerizable monomer (A) (hereinafter, referred to simply as a "(A) component") in the present invention, a polymerizable monomer that can be used in dental cement can be used without any restrictions, but a radically polymerizable monomer is suitably used. Examples of a radical polymerizable unsaturated group of the radically polymerizable monomer include a (meth)acryloyl-based group such as a (meth)acryloyl group, a (meth)acryloyloxy group, a (meth)acryloyl amino group, and a (meth)acryloylthio group, a vinyl group, an allyl group, and a styryl group.

From the viewpoints of polymerizability and safety for living bodies, as the radically polymerizable group, a (meth)acrylic acid ester-based radically polymerizable monomer is suitably used. Specific examples thereof include a monofunctional polymerizable monomer such as ethylhexyl (meth)acrylate, 3-hydroxypropyl (meth)acrylate, and glycidyl(meth)acrylate, a bifunctional polymerizable monomer such as ethylene glycol di(meth)acrylate, diethylene glycol di(meth)acrylate, triethylene glycol di(meth)acrylate, butylene glycol di(meth)acrylate, propylene glycol di(meth)acrylate, 1,6-hexanediol di(meth)acrylate, 1,9-nonanediol di(meth)acrylate, 2,2-bis[4-(3-methacryloyloxy)-2-hydroxypropoxyphenyl]propane, 2,2'-bis(4-methacryloxypolyethoxyphenyl) propane, and 1,6-bis (ethyloxycarbonylamino)trimethylhexane, a trifunctional polymerizable monomer such as trimethylolpropane tri(meth)acrylate and pentaerythritol tri(meth)acrylate, and a tetrafunctional polymerizable monomer such as pentaerythritol tetra(meth)acrylate, ditrimethylolpropane tetra(meth)acrylate, and pentaerythritol hexa(meth)acrylate.

Further, of the above-mentioned acidic groupfree polymerizable monomers, it is favorable to include a two- or higher-functional polymerizable monomer from the viewpoint of mechanical strength.

In the present invention, the above-mentioned polymerizable monomer (A) may be used alone, or two or more types of polymerizable monomers may be combined. Further, a plurality of types of polymerizable monomers having different numbers of functional groups may be combined.

### (c) Filler (B)

The filler (B) (hereinafter, referred to simply also as a "(B) component") according to the present invention is mixed in the amount of 50 to 500 parts by mass, favorably 150 to 400 parts by mass with respect to 100 parts by mass of the polymerizable monomer for the purpose of improving the strength of cement and suppressing shrinkage during polymerization, and of adjusting the viscosity (operability) before the cement is cured. In the case where the amount of the filler (B) is less than 50 parts by mass, sufficient strength as cement cannot be obtained. In the case where the filler (B) is mixed in the amount exceeding 500 parts by mass, the viscosity increases, resulting in poor operability such as heavy mixing, or the cement becomes thick, leading to poor compatibility with the prosthesis, in some cases.

As the filler (B), one or more of selected from the group consisting of an inorganic filler, an organic filler, and an inorganic-organic composite filler can be used as appropriate.

Specific examples of the organic filler used in the present invention include polymethyl (meth)acrylate, polyethyl (meth)acrylate, a noncrosslinked polymer such as a methyl (meth)acrylate·ethyl (meth)acrylate copolymer, a methyl (meth)acrylate·butyl (meth)acrylate copolymer, and a methyl (meth)acrylate·styrene copolymer, and a (meth)acrylate polymer such as a methyl (meth)acrylate·ethylene glycol di(meth)acrylate copolymer, a methyl (meth)acrylate·triethylene glycol di(meth)acrylate copolymer, and a copolymer of methyl (meth)acrylate and a butadiene monomer. Further, a mixture of two or more of these can also be used.

Specific examples of the inorganic filler used in the present invention include quartz, silica, silica-titania, silica-zirconia, lanthanum glass, barium glass, strontium glass, sodium fluoride, ytterbium fluoride, calcium carbonate, aluminum silicate, and fluoroaluminosilicate glass. Note that two or more of these can also be used in combination.

Further, an inorganic-organic composite filler can also be suitably used. The method of forming a composite is not particularly limited, and the composite may be either solid or porous. From the viewpoint of the mechanical strength of the cured body, it is favorable to use an organic-inorganic composite filler in which the surface of inorganic aggregated particles is coated with an organic polymer and has a pore as described in the specification of WO 2013/039169.

By treating the above-mentioned inorganic filler or inorganic-organic composite filler with a surface treatment agent typified by a silane coupling agent, it is possible to improve the affinity with a polymerizable monomer, the dispersibility in a polymerizable monomer, and the mechanical strength and water resistance of the cured body. Such a surface treatment agent is not limited, and the surface treatment method using these only needs to be performed in accordance with known methods.

The particle size and shape of the filler (B) are selected as appropriate and used, but the average particle size is typically 0.001 to 50 µm, particularly favorably 0.001 to 10 µm from the viewpoint of compatibility with the prosthesis.

### (d) Chemical polymerization initiator (C)

In the dental cement according to the present invention, the chemical polymerization initiator (C) including the hydroperoxide (c1) (hereinafter, referred to simply as a "(c1) component") and the specific thiourea compound (c2) represented by the general formula (2) (hereinafter, referred simply as a "(c2) component") is used. The (c1) component functions as an oxidizing agent component and the (c2) component functions as a reducing agent. These two agents react with each other to generate radicals, curing the dental cement according to the present invention. Note that as described above, the chemical polymerization initiator (C) used in the dental cement according to the present invention may include another chemical polymerization initiator within a range that does not impair the effect of the present invention, but does not include, as an arbitrary component, the non-contained aromatic amine that functions as a reducing agent. The components (c1) and (c2) will be described below in detail.

### ·(c1) component

As the (c1) component, a hydroperoxide compound that functions as an oxidizing agent can be used without particular restrictions. Specific examples of the hydroperoxide compound that can be suitably used include 1,1,3,3-tetramethylbutylhydroperoxide, p-menthane hydroperoxide, diisopropylbenzene hydroperoxide, cumene hydroperoxide, t-butyl hydroperoxide, and t-amyl hydroperoxide. Of these, 1,1,3,3-tetramethylbutylhydroperoxide and cumene hydroperoxide can be suitably used from the viewpoints of safety, reactivity, and preservation stability.

The content of the (c1) component in the dental cement according to the present invention is 0.2 to 3.5 parts by mass, favorably 0.25 to 3 parts by mass, with respect to 100 parts by mass of the (A) component from the viewpoints of a chemical polymerization reaction rate and preservation stability. In the case where the content of the (c1) is less than 0.2 parts by mass, radical generation by the chemical reaction is insufficient, resulting in a curing failure and reduced strength of the final cured body after curing. In the case where the content of the (c1) exceeds 3.5 parts by mass, the content of the (A) component relatively decreases, resulting in reduced mechanical strength of the final cured body after curing. Further, radical generation due to decomposition of the (c1) component makes cement gelation more likely and reduces the preservation stability.

### ·(c2) component

The (c2) component functions as a reducing agent and generates radicals through its reaction with the (c1) component, thereby curing the dental cement according to the present invention. Further, the (c2) component also acts as a chain transfer agent and is presumed to suppress the polymerization reaction rate during light irradiation. As a result, it is presumed that adding an appropriate amount of the (c2) component prevents the light irradiation time during which the semi-cured state when removing the excess cement is achieved with photopolymerization from being reduced even if the addition amount of the photopolymerization initiator is large.

The (c2) component is not particularly limited as long as it is the compound represented by the general formula (2). However, in the general formula (2), it is favorable that R¹ and R² each represent a hydrogen atom and R³ represents an alkyl group having 1 to 3 carbon atoms, a pyridyl group, or an aryl group. Examples of such a specific thiourea compound include N-acetylthiourea, 2-pyridylthiourea, and N-benzoylthiourea. From the viewpoints of reactivity as a chemical polymerization initiator and preservation stability, 2-pyridylthiourea and N-benzoylthiourea are particularly suitable. The (c2) component may be used alone, or two or more types of (c2) components may be combined as necessary.

The content of the (c2) component in the dental cement according to the present invention is 0.4 to 1.5 parts by mass, favorably 0.5 to 1.2 parts by mass, with respect to 100 parts by mass of the (A) component, from the viewpoints of the chemical polymerization reaction rate and preservation stability. In the case where the content of the (c2) component is less than 0.4 parts by mass, the effect of the (c2) component as a chain transfer agent cannot be sufficiently achieved, resulting in an excessively high light curing rate. This shortens the light irradiation time in which the semi-cured state is achieved during removal of excess cement. Further, radical generation by a chemical reaction is insufficient, resulting in a curing failure and reduced strength of the cured body under the chemical curing condition. Meanwhile, in the case where the content of the (c2) component exceeds 1.5 parts by weight, the effect of the (c2) component as a chain transfer agent becomes excessive, curing becomes insufficient when the light irradiation intensity is weak and the irradiation time is short, and the removability of excess cement is reduced. Further, the chemical curing rate becomes too high, there is not sufficient time to be able to operate the dental cement.

### (e) Photopolymerization initiator (D)

In the dental cement according to the present invention, the photopolymerization initiator (D) including the α-diketone (d1) (hereinafter, referred to simply also as a "(d1) component"), and the photopolymerization accelerator (d2) (hereinafter, referred to simply as a "(d2) component") is used. These components will be described below.

### ·(d1) component

As the (d1) component, an α-diketone that functions as a photopolymerization initiator can be used without particular restrictions. Examples of the α-diketone that can be suitably used include benzyl, camphorquinone, p,p'-dimethoxybenzyl, p,p'-dictrenequinone, 3,4-phenanthrenequinone, and 9,10-phenanthrenequinone. These α-diketones (d1) can be used alone, or two or more types of α-diketones (d1) can be used in combination. From the viewpoints of preservation stability and the activity as a photopolymerization initiator, camphorquinone is particularly favorably used.

The content of the (d1) component in the dental cement according to the present invention is 0.3 to 0.8 parts by mass, favorably 0.4 to 0.6 parts by mass, with respect to 100 parts by mass of the (A) component, from the viewpoints of removability of excess cement with light curing and the strength of the cured body with light curing. In the case where the content of the (d1) component is less than 0.3 parts by mass, the strength of the dental cement cured with light irradiation is insufficient. Meanwhile, the content of the (d1) component exceeds 0.8 parts by mass, the polymerization rate during light irradiation is too high, resulting in a shorter range of the light irradiation time in which favorable removability of the excess cement can be achieved and poorer removability of the excess cement.

In the dental cement according to the present invention, in order to achieve favorable removability of the excess cement with light curing, it is necessary to set the ratio of the content of the (c2) component and the content of the (d1) component to be within a specific range. Specifically, when the content of the (c2) component is defined as y parts by mass and the content of the (d1) component is defined as x parts by mass, x/y = 0.3 to 1.9, favorably 0.33 to 1.5. In the case where x/y is less than 0.3, the ratio of the (c2) component to the (d1) component becomes too large, and thus, a semi-cured state is not achieved when the light intensity is weak and the light irradiation time is short, resulting in poorer removability of the excess cement. Meanwhile, in the case where x/y exceeds 1.9, the effect of the (c2) component as a chain transfer agent cannot be sufficiently achieved, and the range of the light irradiation time in which favorable removability of the excess cement can be achieved is shortened.

### ·(d2) component

The (d2) component is a component that is used in combination with the (d1) component to promote radical generation. In the dental cement according to the present invention, it is favorable to use tertiary amines (however, excluding the non-contained aromatic amine). In particular, from the viewpoints of reactivity and preservation stability, it is more favorable to use a compound in which an aromatic group is directly substituted with a nitrogen atom. Examples of the photopolymerization accelerator include N,N-dimethylaniline, N,N-diethylaniline, N,N-di-n-butylaniline, N,N-dibenzylaniline, N,N-dimethyl-p-toluidine, N,N-dimethyl-m-toluidine, N,N-diethyl-p-toluidine, p-bromo-N,N-dimethylaniline, m-chloro-N,N-dimethylaniline, p-dimethylaminobenzaldehyde, p-dimethylaminoacetophenone, p-dimethylamino benzoic acid ethyl ester, p-dimethylamino benzoic acid amino ester, N,N-dimethyl anthranilic acid methyl ester, p-dimethylaminostyrene, N,N-dimethyl-3,5-xylidine, 4-dimethyl aminopyridine, N,N-dimethyl-α-naphthylamine, and N,N-dimethyl-β-naphthylamine. These photopolymerization accelerators may each be used alone, or different types of these may be mixed and used. Of these, p-dimethylamino benzoic acid ethyl ester is suitably used.

Note that these tertiary amines include those that function as reducing agents of the chemical polymerization initiator. However, even if such tertiary amines are used, they function as photopolymerization accelerators when light irradiation is performed immediately after the dental cement according to the present invention is prepared.

The content of the (d2) component in the dental cement according to the present invention is 0.3 to 0.8 parts by mass, favorably 0.4 to 0.6 parts by mass, with respect to 100 parts by mass of the (A) component from the viewpoints of the removability of the excess cement with light curing and the strength of the cured body with light curing. In the case where the content of the (d2) component is less than 0.3 parts by mass, the strength of the dental cement cured with light irradiation is insufficient. Meanwhile, in the case where the content of the (d2) component exceeds 0.8 parts by mass, the polymerization rate during light irradiation becomes too large, resulting in a shorter range of the light irradiation time in which favorable removability of the excess cement can be achieved and poorer removability of the excess cement.

### (f) Other components

In the dental cement according to the present invention, an additive can be mixed as an arbitrary component within a range that does not impair the performance. Examples of such an additive include a polymerization inhibitor such as dibutylhydroxytoluene and hydroquinone monomethyl ether, a chain transfer agent, an antioxidant, a pigment, a dye, an ultraviolet absorber, and a thickener.

### 3. Details of dental cement preparation kit

The dental cement preparation kit according to the present invention includes a first agent and a second agent packaged separately, the dental cement according to claim 1 being prepared by mixing the two agents.

Specifically, the kit according to the present invention having the above features includes a combination of a first agent and a second agent packaged in a state where they cannot come into physical contact with each other, the first agent including the first partial composition, the second agent including the second partial composition, the dental cement according to the present invention being prepared by mixing the two agents (two partial compositions) at the time of use. In the dental cement according to the present invention, chemical polymerization starts due to the coexistence of the (c1) component and the (c2) component. Therefore, in the kit according to the present invention, it is necessary to package the (c1) component and the (c2) component separately to ensure stable preservation. Further, the (d1) component is blended in the agent including the (c2) component from the viewpoint of preservation stability, because there is a possibility that the (d1) component is decomposed due to the coexistence with the (c1) component. For this reason, the first agent includes the (c2) component and the (d1) component, the second agent includes the (c1) component, and the first agent and the second agent are packaged separately such that these components do not react with each other before use. Further, the polymerizable monomer (A) is divided and included in both the first agent and the second agent. Note that the photopolymerization accelerator (d2) and the filler (B) may be blended in either the first agent or the second agent.

That is, when the agent that includes the thiourea compound (c2) is the first agent, the first agent includes part of the polymerizable monomer (A), the thiourea compound (c2), and the α-diketone (d1), and does not include the hydroperoxide (c1), the second agent includes the remainder of the polymerizable monomer (A) and the hydroperoxide (c1), and does not include the thiourea compound (c2) and the α-diketone (d1), and the filler (B) and the photopolymerization accelerator (d2) are included in either the first agent or the second agent or divided and included in both the first agent and the second agent.

However, since the (d2) component may be decomposed when coexisting with the (c1) component, although not to the same extent as the (d1) component, it is favorable that the (d2) component is included in the agent that includes the (c2) component, and the following packaging method is favorably adopted from the viewpoint of preservation stability.
First agent: part of the (A) component, part of the (B)component, the (c2) component, the (d1) component, and the (d2) component
Second agent: remainder of the (A) component, remainder of the (B)component, and the (c1) component.

Note that the phrase "packaged in a state where they cannot come into physical contact with each other" means a state where the first agent and another agent are separated by a barrier member (packaging member) that prevents molecular diffusion between them and packaged. As the barrier member (packaging member), a resin or the like that is suitably used as a material of a container or bag is generally used. Typical examples of the "state where they cannot come into physical contact with each other" include a state where one type of composition is preserved while being hermetically sealed in a container blocking from outside air and outside light. Examples of specific packing forms include a form of being deposited in a container such as a bottle, a tube, and a syringe. As a method of preparing the dental cement according to the present invention using the kit according to the present invention, for example, i) a method of applying an appropriate amount of the first agent and the second agent onto mixing paper and mixing them sing a spatula, ii) a method of simultaneously extruding the first agent and the second agent from a syringe with a mixing chip connected to the tip thereof in the case where the first agent and the second agent are paste-like agents, or iii) a method of taking the first agent and the second agent into the same mixing dish in the case where the first agent and the second agent are liquid can be adopted.

The composition of each of the first agent and the second agent is determined basically such that when the two agents are mixed in equal amounts, i.e., when the mixing ratio of the first agent and the second agent (the amount of first agent / the amount of second agent) is 1/1, or when the mixing percentage of the first agent and the second agent (100 × the amount of first agent / the amount of second agent) is 100%, the composition of the dental cement according to the present invention is obtained. Then, by weighing and mixing each component in accordance with the composition determined in this way, the first agent and the second agent can be easily prepared. Note that the above "equal amounts" typically mean equal amounts based on mass. However, when the composition is liquid, the above "equal amounts" may be equal amounts based on volume.

However, in actual use, i.e., when the first agent and the second agent are mixed to prepare the dental cement according to the present invention, the mixing ratio cannot be maintained at strictly 1/1 in some cases. In this case, it is favorable that a mixing ratio other than 1/1 (hereinafter, referred to also as a "designated mixing ratio"), the dental cement according to the present invention having a desired composition is obtained. The designated mixing ratio (this value expressed as percentage is referred to also as a "designated mixing percentage") only needs to be appropriately determined within a range where polymerization activity and operability are not significantly impaired. However, from practical considerations such as ease of handling and product packaging, regarding the designated mixing ratio (percentage), the mixing ratio (first agent/second agent) based on mass (or volume) is favorably within a range of 1/5 to 5/1 (mixing percentage: 20% to 500%), more favorably 1/3 to 3/1 (mixing percentage: 33% to 300%).

The designated mixing ratio (percentage) can be displayed on a mixing ratio (percentage) information display medium. As this mixing ratio (percentage) information display medium, for example, i) a product package formed of a paper box or the like, ii) a product instruction manual provided as a paper medium and/or electronic data, iii) a container (a bottle, a syringe, a packaging bag, or the like) for preserving each of the first agent and the second agent in a sealed condition, iv) a product catalog provided as a paper medium and/or electronic data, or v) a message transmitted to a product user by an email or postal mail separately from a product can be used. Further, the designated mixing percentage may be provided to the product user in an aspect that can be recognized by the product user other than the aspects shown in the above i) to v).

In the case of using the kit according to the present invention, since the mixed composition (dental cement according to the present invention) obtained by mixing the first partial composition (first agent) and the second partial composition (second agent) includes all types of chemical polymerization initiator (C) and polymerizable monomer (A), it is polymerized and cured rapidly or within a predetermined operation margin time. As a result, a cured body can be obtained. As the method of polymerizing and curing the mixed composition, a known method can be adopted. For example, it only needs to apply the mixed composition to the part to be cured and allow it to stand. In this case, by maintaining the applied mixed composition in the temperature range of 10 to 37°C, the mixed composition can be sufficiently cured.

### (Examples)

Although the present invention will be specifically described below by way of Examples, the present invention is not limited to these Examples.
1. First, the abbreviations of the substances used in the components according to Examples and Comparative Examples and the compositions including these will be described below.

### <Polymerizable monomer (A)>

Bis-GMA; 2,2-bis[4-(3-methacryloyloxy)-2-hydroxypropoxyphenyl]propane
3G; triethylene glycol dimethacrylate
D-2.6E; 2,2'-bis(4-methacryloxypolyethoxyphenyl) propane.

### <Filler (B)>

F1; silica zirconia filler with an average particle diameter of 3 µm
F2; silica zirconia filler with an average particle diameter of 0.2 µm.

### <Chemical polymerization initiator (C)>

·Hydroperoxide (c1)
   PO1; 1,1,3,3-tetramethylbutylhydroperoxide
   PO2; cumene hydroperoxide
·Specific thiourea compound (c2)
   BzTU; N-benzoylthiourea
   PyTU; 2-pyridylthiourea.

### <Chemical polymerization initiator other than (c1) component and (c2) component>

BPO; benzoyl peroxide
DMPT; N,N-dimethyl-p-toluidine
DEPT;N,N-di(1-hydroxyethyl)-p-toluidine (that is the non-contained aromatic amine)

### <Photopolymerization initiator (D)>

·α-diketone (d1)
   CQ; camphorquinone
·Photopolymerization accelerator (d2)
   DMBE; p-dimethylamino benzoic acid ethyl ester.

### <Others>

BHT; dibutylhydroxytoluene.

2. Next, dental cements according to Examples 1 to 15 and Comparative Examples 1 to 10 were prepared by the following method and evaluated.

### Example 1

A first agent including a first partial composition was prepared by dissolving PyTU: 1 part by mass, CQ: 0.5 parts by mass, DMBE: 0.5 parts by mass, and BHT: 0.15 parts by mass in the polymerizable monomer (A): 50 parts by mass including BisGMA: 6 parts by mass, 3G: 19 parts by mass, and D-2.6E: 25 parts by mass and then mixing the filler (B): 143 parts by mass including F1: 57 parts by mass and F2: 86 parts by mass therein. Further, a second agent including a second partial composition was prepared by dissolving PO1: 2.5 parts by mass and BHT: 0.3 parts by mass in the polymerizable monomer (A): 50 parts by mass including BisGMA: 6 parts by mass, 3G: 19 parts by mass, and D-2.6E: 25 parts by mass and then mixing the filler (B):143 parts by mass including F1: 57 parts by mass and F2: 86 parts by mass.

Dental cement was prepared by mixing the first agent and the second agent prepared in this way in equal amounts, and the removability of the excess cement and the bending strength of the cured body were evaluated by the method described below. As a result, the removability of the excess cement was A with the irradiation time of 1 to 3 seconds in the case of a light intensity: 300 mW/cm², and A with the irradiation time of 3 to 4 seconds and B with the irradiation time of 5 seconds in the case of a light intensity: 1200 mW/cm², and the bending strength of the cured body was 152 MPa.

### <Method of evaluating removability of excess cement>

A cow tooth extracted within 24 hours of slaughter was ground under water using P600 waterproof abrasive paper, approximately 1 cm² of the enamel was ground to a flat surface parallel to the lip surface, and it was incubated in a constant temperature bath at 37°C for 2 hours. After the first agent and the second agent were mixed in equal amounts and mixed for 30 seconds to prepare dental cement, the cow tooth was taken out of the constant temperature bath immediately, 15 mg of the dental cement was placed on the enamel surface, a 2 mm square aluminum plate was pressed against the placed cement, and the cement was caused to protrude around the aluminum plate (the protruding cement is the excess cement), thereby preparing a test sample.

The excess cement obtained in this way was irradiated with light by adjusting the distance between the excess cement and the tip of the light irradiator such that the light intensity was 300 mW/cm² or 1200 mW/cm².

The light irradiation was started immediately after the aluminum plate was pressed against the placed cement to cause the excess cement to protrude during the preparation of the test sample, and was performed for 1 to 5 seconds with the duration increased by 1 second at each interval. For each of the test samples irradiated with light for the above number of seconds, a dental short needle was inserted into the excess cement immediately and attempts were made to remove it. At that time, the degree of the excess cement that could be removed by adhering to the dental short needle was evaluated on the basis of the following criteria.
A: The hardness of the excess cement is appropriate, the short needle can be smoothly inserted into the excess cement, and the excess cement can be effectively removed in a large chunk.
B: The hardness of the excess cement is slightly softer than the appropriate hardness, the excess cement can be peeled off from the aluminum plate, but the excess cement cannot be removed in a large chunk using the short needle. Alternatively, the excess cement is cured too much and slightly harder than the appropriate hardness, and thus, the short needle cannot be inserted into the excess cement unless a lot of force is used.
C: The hardness of the excess cement is significantly softer than the appropriate hardness, and thus, the fluidity of the excess cement is high and the excess cement cannot be removed in a chunk even if the short needle is inserted into it. Alternatively, the excess cement is substantially completely cured and the short needle cannot be inserted into it.

### <Evaluation of bending strength of cured body>

A paste mixed for 10 seconds was deposited into a mold with a square column-shaped hole of 2 × 2 × 25 mm, sandwiched between polyethylene films and slide glasses on both sides, and pressed together using a clip. One side was irradiated with light from above the film and the slide glass at five points each for 20 seconds, and the back surface was irradiated with light similarly. The light intensity at this time was set to 500 mW/cm² by adjusting the distance between the dental cement and the tip of the light irradiator. After taking the cured body taken out of the mold, it was ground with P320 waterproof abrasive paper and immersed in water at 37°C overnight to obtain a test piece. The three-point bending-shear strength of this test piece was measured at a crosshead speed of 1.0 mm/min using a universal testing machine (AG-I, manufactured by Shimadzu Corporation). Five test pieces were prepared, and the average was taken as the bending strength.

### Examples 2 to 15 and Comparative Examples 1 to 10

First agents and second agents were prepared in the same manner as that in Example 1 except that the types and contents of the polymerization initiator and the polymerization inhibitor were changed as shown in Table 1 and Table 2.

Note that the parts by mass (numerical values in parentheses) of each component shown in Table 1 and Table 2 represents the parts by mass with respect to the polymerizable monomer (A): 50 parts by mass of the first agent for the first agent composition and parts by mass with respect to the polymerizable monomer (A): 50 parts by mass of the second agent for the second agent composition. That is, the components of the first agent and the second agent other than the polymerization initiator and the polymerization inhibitor in each of Examples and Comparative Examples are common, and the compositions of the first agent and the second agent in each of Examples and Comparative Examples are obtained by mixing the base composition having the following composition with the compounds shown in Table 1. For example, as shown in Table 1, the content of each component in the first agent in Example 1 is PyTU: 1 part by mass, CQ: 0.5 parts by mass, DMBE: 0.5 parts by mass, and BHT: 0.15 parts by mass. Further, "↑" in Table means the same as above.

### ·Composition of base composition

Polymerizable monomer (A)
   BisGMA: 6 parts by mass
   3G: 19 parts by mass
   D-2.6E: 25 parts by mass
Filler (B)
   F1: 57 parts by mass
   F2: 86 parts by mass

The first agent and the second agent prepared in this way were mixed in equal amounts to prepare dental cement, and the removability of the excess cement and the bending strength of the cured body were evaluated in the same manner as that in Example 1. The results are shown in Table 3 and Table 4.

**(Table 1)**

| | First agent composition | | | | Second agent composition | | Ratio x/y of (d1)/(d2) |
|---|---|---|---|---|---|---|---|
| | (c2)Specific thiourea compound | (d1) α-diketone | (d2)Photopoly -merization accelerator | Other components | (c1)Hydro -peroxide | Other components | |
| Example 1 | PyTU(1) | CQ(0.5) | DMBE(0.5) | BHT(0.15) | PO1(2.5) | BHT(0.3) | 0.50 |
| Example 2 | BzTU(1) | ↑ | ↑ | ↑ | PO2(2.5) | ↑ | 0.50 |
| Example 3 | PyTU(0.4) | ↑ | ↑ | ↑ | PO1(2.5) | ↑ | 1.25 |
| Example 4 | PyTU(1.5) | ↑ | ↑ | ↑ | ↑ | ↑ | 0.33 |
| Example 5 | PyTU(1) | CQ(0.3) | ↑ | ↑ | ↑ | ↑ | 0.30 |
| Example 6 | ↑ | CQ(0.8) | ↑ | ↑ | ↑ | ↑ | 0.80 |
| Example 7 | ↑ | CQ(0.5) | DMBE(0.3) | ↑ | ↑ | ↑ | 0.50 |
| Example 8 | ↑ | ↑ | DMBE(0.8) | ↑ | ↑ | ↑ | 0.50 |
| Example 9 | PyTU(1.3) | CQ(0.4) | DMBE(0.5) | ↑ | ↑ | ↑ | 0.31 |
| Example 10 | PyTU(0.4) | CQ(0.75) | ↑ | ↑ | ↑ | ↑ | 1.88 |
| Example 11 | PyTU(1) | CQ(0.5) | ↑ | ↑ | PO1(0.2) | ↑ | 0.50 |
| Example 12 | ↑ | ↑ | ↑ | ↑ | PO1(0.3) | ↑ | 0.50 |
| Example 13 | ↑ | ↑ | ↑ | ↑ | PO1(3) | ↑ | 0.50 |
| Example 14 | ↑ | ↑ | ↑ | ↑ | PO1(3.5) | ↑ | 0.50 |
| Example 15 | ↑ | ↑ | ↑ | - | PO1(2.5) | - | 0.50 |

**(Table 2)**

| | First agent composition | | | | Second agent composition | | Ratiox/y of (d1)/(2) |
|---|---|---|---|---|---|---|---|
| | (c2)Specific thiourea compound | (d1) α-diketone | (d2)Photopoly -merization accelerator | Other components | (c1)Hydro -peroxide | Other components | |
| Comparative Example 1 | PyTU(1.2) | CQ(0.3) | DMBE(0.3) | BHT(0.15) | PO1(2.5) | BHT(0.3) | 0.25 |
| Comparative Example 2 | PyTU(0.4) | CQ(0.8) | DMBE(0.5) | ↑ | ↑ | ↑ | 2.00 |
| Comparative Example 3 | PyTU(0.3) | CQ(0.5) | DMBE(0.75) | ↑ | ↑ | ↑ | 1.67 |
| Comparative Example 4 | PyTU(1.6) | CQ(0.3) | DMBE(0.3) | ↑ | ↑ | ↑ | 0.38 |
| Comparative Example 5 | ↑ | CQ(0.2) | ↑ | ↑ | ↑ | ↑ | 0.33 |
| Comparative | PyTU(0.7) | CQ(0.9) | DMBE(0.8) | ↑ | ↑ | ↑ | 1.29 |
| Comparative Example 7 | PyTU(0.6) | CQ(0.3) | DMBE(0.2) | ↑ | ↑ | ↑ | 0.50 |
| Comparative Example 8 | PyTU(0.4) | CQ(0.75) | DMBE(0.9) | ↑ | ↑ | ↑ | 1.88 |
| Comparative Example 9 | - | CQ(0.5) | DMBE(0.5) | BHT(0.15) | - | BPO(3.5) | - |
| | | | | DEPT(2.5) | | | |
| | | | | DMPT(0.5) | | BHT(0.3) | |
| Comparative Example 10 | PyTU(1) | CQ(0.5) | DMBE(0.3) | BHT(0.15) | PO1(2.5) | BHT(0.3) | 0.50 |
| | | | | DEPT(2.5) | | | |

**(Table 3)**

| | Bending strength /MPa | Removability of excess cement | | | | | |
|---|---|---|---|---|---|---|---|
| | | 300mW/cm² | | | 1200mW/cm² | | |
| | | 1 second | 2 second | 3 second | 3 second | 4 second | 5 second |
| Example 1 | 152 | A | A | A | A | A | B |
| Example 2 | 155 | A | A | A | A | A | B |
| Example 3 | 149 | A | A | A | A | A | B |
| Example 4 | 147 | A | A | A | A | A | A |
| Example 5 | 137 | A | A | A | A | A | A |
| Example 6 | 151 | A | A | A | A | A | B |
| Example 7 | 134 | A | A | A | A | A | A |
| Example 8 | 152 | A | A | A | A | A | B |
| Example 9 | 139 | A | A | A | A | A | A |
| Example 10 | 155 | A | A | A | A | A | B |
| Example 11 | 137 | A | A | A | A | A | B |
| Example 12 | 150 | A | A | A | A | A | B |
| Example 13 | 154 | A | A | A | A | A | B |
| Example 14 | 138 | A | A | A | A | A | B |
| Example 15 | 154 | A | A | A | A | A | B |

**(Table 4)**

| | Bending strength /MPa | Removability of excess cement | | | | | |
|---|---|---|---|---|---|---|---|
| | | 300mW/cm² | | | 1200mW/cm² | | |
| | | 1 second | 2 second | 3 second | 3 second | 4 second | 5 second |
| Comparative Example 1 | 132 | C | A | A | A | A | A |
| Comparative Example 2 | 151 | A | A | A | A | B | **C** |
| Comparative Example 3 | 153 | A | A | A | A | **C** | **C** |
| Comparative Example 4 | 131 | C | A | A | A | A | A |
| Comparative Example 5 | 127 | A | A | A | A | A | A |
| Comparative Example 6 | 155 | A | A | A | A | B | **C** |
| Comparative Example 7 | 125 | A | A | A | A | A | A |
| Comparative Example 8 | 156 | A | A | A | A | B | **C** |
| Comparative Example 9 | 150 | C | C | A | A | A | A |
| Comparative Example 10 | 148 | C | C | A | A | A | A |

Examples 1 to 15 are compositions that satisfy the configuration of the present invention and exhibited favorable removability of the excess cement and a high bending strength.

On the other hand, in Comparative Example 1 in which x/y is less than0.3 when the content of the (d1) component is x parts by mass and the content of the (c2) component is y parts by mass and Comparative Example 4 in which the content of the (c2) component exceeds 1.5 parts by mass with respect to 100 parts by mass of the (A) component, the effect of the (c2) component as a chain transfer agent became excessive, resulting in poor removability of the excess cement in the case where the light intensity is weak and the irradiation time is short.

Further, in Comparative Example 2 in which x/y exceeds 1.9, Comparative Example 3 in which the content of the (c2) component with respect to 100 parts by mass of the (A) component is less than 0.4, Comparative Example 6 in which the content of the (d1) component with respect to 100 parts by mass of the (A) component exceeds 0.8, and Comparative Example 8 in which the content of the (d2) component with respect to 100 parts by mass of the (A) component exceeds 0.8, the effect of the (c2) component as a chain transfer agent could not be sufficiently achieved, resulting in poor removability of the excess cement in the case where the light intensity is strong and the irradiation time is long.

Further, in Comparative Example 5 in which the content of the (d1) component with respect to 100 parts by mass of the (A) component is less than 0.3 and Comparative Example 7 in which the content of the (d2) component with respect to 100 parts by mass of the (A) component is less than 0.3, a low bending strength was exhibited due to the insufficient photopolymerization initiator.

Further, in Comparative Example 9 and Comparative Example 10 in which the non-contained aromatic amine is used, which correspond to the dental cement disclosed in Patent Literature 1, the photopolymerization reaction was inhibited by the non-contained aromatic amine, resulting in poor removability of the excess cement in the case where the light intensity is weak and the irradiation time is short.

## Claims

1. Dental cement, comprising:
a composition that includes a polymerizable monomer (A), a filler (B), a chemical polymerization initiator (C), and a photopolymerization initiator (D), the dental cement being **characterized in that**
a blending amount of the filler (B) is 50 to 500 parts by mass with respect to 100 parts by weight of the polymerizable monomer (A),
a tertiary aromatic amine represented by the following general formula (1) is not substantially included,
the chemical polymerization initiator (C) includes a hydroperoxide (c1): 0.2 to 3.5 parts by mass and a thiourea compound (c2) represented by the following general formula (2): 0.4 to 1.5 parts by mass,
the photopolymerization initiator (D) includes an α-diketone (d1): 0.3 to 0.8 parts by mass and a photopolymerization accelerator (d2): 0.3 to 0.8 parts by mass, and
a ratio of a content (parts by mass) of the α-diketone (d1) to a content (parts by mass) of the thiourea compound (c2) is 0.3 to 1.9. 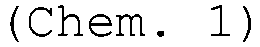 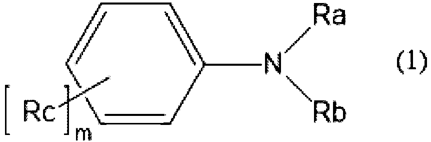 (wherein, Rₐ and R_{b} each represent an alkyl group having 1 to 6 carbon atoms or a substituted alkyl group having 1 to 6 carbon atoms having a hydroxyl group, a nitro group, a sulfonic group, or a halogen atom as a substituent group, which may be different from each other, at least one of Rₐ or R_{b} represents the substituted alkyl group, R_{c} represents an alkyl group having 1 to 4 carbon atoms, and m represents an integer of 0 to 3.) 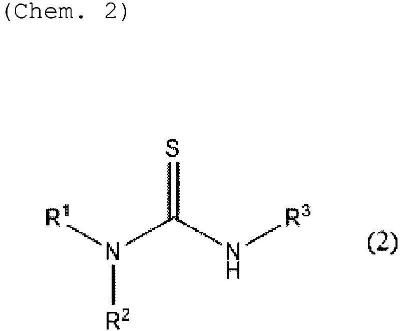 (wherein, R¹, R², and R³ each represent a hydrogen atom, an alkyl group, a cycloalkyl group, an aryl group, a heterocyclic group, an acyl group, an aralkyl group, or an alkenyl group, and R¹ and R² may be bonded to each other to form a ring.)

2. A dental cement preparation kit, comprising:
a first agent and a second agent packaged separately, the dental cement according to claim 1 being prepared by mixing the two agents, the dental cement preparation kit being characterized that
the first agent includes the thiourea compound (c2) and the α-diketone (d1), and
the second agent includes the hydroperoxide (c1).
